# EUROPEAN PATENT APPLICATION

(11) **EP 4 296 348 A1**
(43) Date of publication of application: **27.12.2023**
(21) Application number: 23152500.7
(22) Date of filing: 19.01.2023
(51) Int. Cl.: C12M 1/34, C12M 1/36, G01N 21/65

(54) **CALIBRATION DEVICE, CALIBRATION METHOD, AND CALIBRATION PROGRAM**

(30) Priority: 23.06.2022 JP 2022100959
(71) Applicant: Yokogawa Electric Corporation, Musashino-shi, Tokyo 180-8750 (JP)
(72) Inventor: KOBAYASHI, Wataru, Tokyo, 180-8750 (JP); HARA, Risa, Tokyo, 180-8750 (JP); MURAYAMA, Kodai, Tokyo, 180-8750 (JP); SHIMODA, Souichirou, Tokyo, 180-8750 (JP)
(74) Representative: Zimmermann, Tankred Klaus

(57) **Abstract**

A calibration device (10) generates a data set of a reference sample including spectral data of the reference sample containing a plurality of components and each objective variable determined by a content of each of the components of the reference sample, and trains, by machine learning using the data set of the reference sample, a machine learning model that outputs at least one objective variable among the objective variables of each of the components in response to input of the spectral data.

## Description

### FIELD

The present invention relates to a calibration device, a calibration method, and a calibration program.

### BACKGROUND

Conventionally, there is a prediction technology for predicting process values such as nutrient concentration values in cell culture including animal cells, microorganisms, plant cells, and the like, for example, using a cell culture device (bioreactor). For example, in the above prediction technology, spectral data is acquired from the bioreactor, and offline measurement data of nutrient concentrations of glucose and the like is acquired, using any appropriate analytical method. In the above prediction technology then, the peaks of the spectral data are correlated with offline measurement values of process variables, and chemometric modeling is executed to predict process values such as nutrient concentration values. The related technologies are described, for example, in: Japanese Patent Application Laid-open No. 2020-195370; Japanese Translation of PCT International Application Publication No. 2020-537126; Japanese Patent Application Laid-open No. 2016-128822; Thaddaeus A. Webster, Development of Generic Raman Models for a GS-KOTM CHO Platform Process, Biotechnol. Prog., 2018, Vol. 34; and Hemlata Bhatia, Inline monitoring of amino acids in mammalian cell cultures using raman spectroscopy and multivariate chemometrics models, Eng. Life Sci. 2018, 18, 55-61.

However, with the above prediction techniques, it is difficult to create a highly accurate calibration model in an effective manner, because the predicted values may deviate significantly from the actually measured values when conditions of cell cultures vary greatly.

The present invention is designed in view of the aforementioned circumstances, and an object thereof is to create a highly accurate calibration model in an effective manner.

### SUMMARY

According to an aspect of the embodiments, a calibration device includes, a generation unit that generates a data set of a reference sample including spectral data of the reference sample containing a plurality of components and each objective variable determined by a content of each of the components of the reference sample, and a training unit that trains, by machine learning using the data set of the reference sample, a machine learning model that outputs at least one objective variable among the objective variables of each of the components in response to input of the spectral data.

According to an aspect of the embodiments, a calibration method includes, generating a data set of a reference sample including spectral data of the reference sample containing a plurality of components and each objective variable determined by a content of each of the components of the reference sample, and training, by machine learning using the data set of the reference sample, a machine learning model that outputs at least one objective variable among the objective variables of each of the components in response to input of the spectral data.

According to an aspect of the embodiments, a calibration program that causes a computer to execute a process includes, generating a data set of a reference sample including spectral data of the reference sample containing a plurality of components and each objective variable determined by a content of each of the components of the reference sample, and training, by machine learning using the data set of the reference sample, a machine learning model that outputs at least one objective variable among the objective variables of each of the components in response to input of the spectral data.

### BRIEF DESCRIPTION OF THE DRAWINGS

Fig. 1 is a diagram illustrating a configuration example of a calibration system according to an embodiment;
Fig. 2 is a block diagram illustrating configuration examples of devices of the calibration system according to the embodiment;
Fig. 3 is a graph illustrating an example of an estimation result according to the embodiment;
Fig. 4 is a table illustrating an example of an estimation result according to the embodiment;
Fig. 5 is a graph illustrating an example of an estimation result according to the embodiment;
Fig. 6 is a flowchart illustrating an example of a flow of data estimation processing according to the embodiment;
Fig. 7 is a flowchart illustrating an example of a flow of calibration model construction processing 1 according to the embodiment;
Fig. 8 is a flowchart illustrating an example of a flow of calibration model construction processing 2 according to the embodiment;
Fig. 9 is a table illustrating an example of evaluation of the calibration model construction processing 2 according to the embodiment; and
Fig. 10 is a diagram for describing an example of a hardware configuration.

### DESCRIPTION OF EMBODIMENTS

Hereinafter, a calibration device, a calibration method, and a calibration program according to an embodiment of the present invention will be described in detail with reference to the accompanying drawings. Note that the present invention is not limited by the embodiment described below.

### Embodiment

Hereinafter, the configuration of the calibration system according to the embodiment, the configurations of the calibration device and the like, and flows of processing will be described in order, and effects of the embodiment will be described in the end.

### 1. Configuration of Calibration System 100

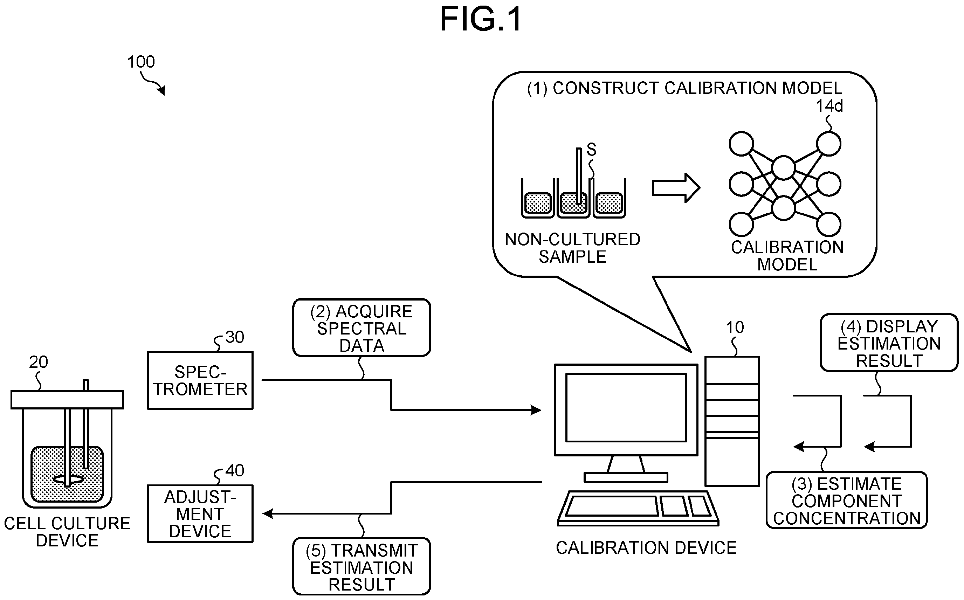

The configuration of a calibration system 100 according to the embodiment will be described in detail by referring to Fig. 1. Fig. 1 is a diagram illustrating a configuration example of the calibration system 100 according to the embodiment. Hereinafter, a configuration example of the entire calibration system 100, processing of the calibration system 100, and the effects of the calibration system 100 will be described in this order.

### 1-1. Configuration Example of Entire Calibration System 100

The calibration system 100 includes a calibration device 10, a cell culture device 20, a spectrometer 30, and an adjustment device 40. The calibration system 100 illustrated in Fig. 1 may include a plurality of calibration devices 10, a plurality of cell culture devices 20, a plurality of spectrometers 30, or a plurality of adjustment devices 40. The calibration device 10 may be integrated with one or more of the cell culture device 20, the spectrometer 30, and the adjustment device 40. Hereinafter, the calibration device 10, the cell culture device 20, the spectrometer 30, and the adjustment device 40 will be described in this order.

### 1-1-1. Calibration Device 10

The calibration device 10 is communicatively connected to the spectrometer 30, and acquires spectral data of a cell culture medium in the cell culture device 20. The calibration device 10 is communicatively connected to the adjustment device 40, and transmits, to the adjustment device 40, an estimation result of concentrations of the components of the cell culture medium in the cell culture device 20.

### 1-1-2. Cell Culture Device 20

The cell culture device 20 includes a cell culture tank that houses therein a cell culture medium containing cells such as animal cells, microorganisms, and plant cells. The cell culture medium housed in the cell culture tank of the cell culture device 20 contains glucose, lactic acid, antibodies, and the like as components. The cell culture device 20 is controllably connected to the spectrometer 30 and the adjustment device 40.

### 1-1-3. Spectrometer 30

The spectrometer 30 is controllably connected to the cell culture device 20, and measures spectral data of the cell culture medium in the cell culture device 20. Although not specifically limited, the spectrometer 30 may be a Raman spectrometer, a near-infrared spectrometer, an infrared spectrometer, an ultraviolet spectrometer, a fluorescence spectrometer, a visible spectrometer, or the like.

### 1-1-4. Adjustment Device 40

The adjustment device 40 is controllably connected to the cell culture device 20, and adjusts the components of the cell culture medium in the cell culture device 20.

### 1-2. Processing of Entire Calibration System 100

The processing of the entire calibration system 100 will be described. Note that the following processing may be executed also in a different order. In addition, some of the following pieces of processing may be omitted.

### 1-2-1. Calibration Model Construction Processing

The calibration device 10 constructs a calibration model 14d using a non-cultured sample S (see (1) in Fig. 1). Here, the non-cultured sample S is a solution in which a measurement-target component and a non-measurement component contained in the cell culture medium are prepared in various concentrations. For example, the calibration device 10 acquires spectral data of the non-cultured sample S measured by a non-cultured sample measurement device (not illustrated), and stores the data in a storage unit 14 to be described later. The calibration device 10 also performs sampling before or after the spectral data measurement of the non-cultured sample S to acquire offline measurement data of an objective variable (e.g., nutrient concentration of glucose or the like) using any appropriate analytical method, and stores the data in the storage unit 14.

The calibration device 10 reads a calibration set from the storage unit 14. Note here that the calibration set is a supervised data set generated from the spectral data of the non-cultured sample S and the offline measurement data of the objective variable. Next, the calibration device 10 searches for a development condition of the calibration model 14d by cross-validation. Note here that the development condition refers to the type of analysis algorithm and parameters for spectral preprocessing, a wavenumber range, a regression coefficient of a regression model, signal processing, and the like. The calibration device 10 then constructs the calibration model 14d based on the result of the condition search. Furthermore, the calibration device 10 stores, in the storage unit 14, the calibration model information that is the derived development condition.

At this time, the calibration device 10 can also create a more accurate calibration model 14d by using a newly acquired data set by operating the cell culture device 20. In other words, the calibration device 10 reads a calibration set and the newly acquired data set from the storage unit 14. At this time, the calibration device 10 acquires the spectral data of the cell culture medium of the cell culture device 20 measured by the spectrometer 30. The calibration device 10 also acquires the objective variable data measured from the cell culture medium of the cell culture device 20 by any appropriate analytical method. The calibration device 10 then searches for the development condition of the calibration model 14d by using the newly acquired data set as a validation set. The calibration device 10 then constructs the calibration model 14d based on the result of the condition search.

### 1-2-2. Spectral Data Acquisition Processing

The calibration device 10 acquires the spectral data of the cell culture medium from the spectrometer 30 connected to the cell culture device 20 (see (2) in Fig. 1). For example, the calibration device 10 acquires the spectral data of the cell culture medium measured by the spectrometer 30 that is a Raman spectrometer.

### 1-2-3. Objective Variable Estimation Processing

The calibration device 10 estimates the objective variable such as the component concentration or the like by inputting the spectral data of the cell culture medium into the calibration model 14d (see (3) in Fig. 1). At this time, the input spectral data is applied to the calibration model information read out from the storage unit 14 to calculate the predicted value (estimation result) of the amount of the measurement target component from the peak intensity.

### 1-2-4. Estimation Result Display Processing

The calibration device 10 displays the estimation result regarding the cell culture medium (see (4) in Fig. 1). For example, the calibration device 10 displays a process variable such as a nutrient concentration value as the measurement target component in the cell culture medium.

### 1-2-5. Estimation Result Transmission Processing

The calibration device 10 transmits the estimation result regarding the cell culture medium to the adjustment device 40 (see (5) in Fig. 1). For example, the calibration device 10 transmits the process variable such as the nutrient concentration value to the adjustment device 40. At this time, the adjustment device 40 optimizes the environment of the cell culture device 20 by adjusting nutrient concentration value and the like.

### 1-3. Effect of Calibration System 100

Hereinafter, problems in the calibration processing of the reference technology will be described, and the effect of the calibration system 100 will be described thereafter.

### 1-3-1. Problems in Calibration Processing of Reference Technology

In the calibration processing of the reference technology, a Raman spectrometer is connected to a cell culture device (bioreactor) to acquire spectral data. Furthermore, in the processing, offline measurement data of nutrient (glucose and the like) concentrations is acquired using any appropriate analytical method. Then, in the processing, a multivariate software package is used to correlate the peaks of the spectral data to the offline measurement values of the process variables. At this time, the processing may also include preprocessing by smoothing or normalization. Then, in the processing, chemometric modeling is executed to predict the process values such as the nutrient concentration values. At this time, in the processing, filtering may also be performed for noise reduction. Furthermore, in the processing, the predicted process values are used to perform Raman real-time analysis and feedback control so as to provide continuous and low-concentration nutrients to the cell culture. The calibration processing described above has the following problems.

### 1-3-1-1. Problem 1

In the calibration processing described above, spectral data acquired by connecting the spectrometer to the cell culture device and concentration data acquired by sampling are used as a model creation data set (calibration set) (n = 7 to 12). In the processing, in order to create a highly accurate calibration model, it is necessary to have a calibration set that satisfies the following conditions: equivalence (similarity) is high with the spectrum (validation set) that is acquired when an actual model is used; the concentration range is covered and the concentration intervals are uniform; all conditions are taken into account and no accidental cross-correlation is observed; and the like. For that, it is necessary to conduct cell culture for a plurality of times under a plurality of conditions.

### 1-3-1-2. Problem 2

With the calibration processing described above, an enormous amount of time and cost are required for creating a model. For example, with the processing, the cost for cell culture of one time (5-L-scale) is about 100000 to 200000 yen, and the culture is conducted for about 1 to 2 weeks.

### 1-3-1-3. Problem 3

While the calibration processing described above requires cell culture with sampling, sampling involves the risk of loss and contamination of the culture medium.

### 1-3-1-4. Problem 4

In the calibration processing described above, the cell culture conditions under which the measurement is performed and the cell culture conditions at the time of creating a model are set to be the same to enable a highly accurate prediction. However, with the processing, there is a risk that the predicted values may deviate significantly from the measured values when the cell culture conditions vary greatly. For example, with the processing, the predicted values may deviate significantly from the measured values in cultures in which the cell type, cell density, medium, feed agent, and the like vary or these are different in combination.

### 1-3-1-5. Problem 5

The calibration processing described above may result in having less robustness of a quantitative model for the cell culture conditions and the device installation conditions.

### 1-3-2. Overview of Calibration System 100

In the calibration system 100, the calibration device 10: generates a supervised data set of a non-cultured sample S containing spectral data and component concentrations of the non-cultured sample S containing a plurality of components; and, by machine learning using the data set, trains the calibration model 14d that is a machine learning model that outputs a target component concentration in response to input of the spectral data. In this case, the calibration device 10 generates the supervised data set by using the non-cultured samples S prepared with various concentrations of components contained in the cell culture medium. The calibration device 10 also acquires spectral data measured by a spectral analysis performed on the cell culture medium, and estimates the concentration of components contained in the cell culture medium based on the result acquired by inputting the spectral data into the trained calibration model 14d.

### 1-3-3. Effect of Calibration System 100

First, the calibration system 100 does not require cell culture with sampling for preparing a calibration set, so that the time and cost for creating a model can be reduced significantly. Secondly, the calibration system 100 eliminates the risk of loss and contamination of the culture medium. Thirdly, the calibration system 100 improves the robustness of the quantitative model for the cell culture conditions and the model can be applied to various culture conditions, because the calibration model 14d is not overtrained since samples with no cross-correlation between components are prepared and used as a calibration set. Fourthly, the calibration system 100 can reduce the number of data pieces in the calibration set compared to conventional cases, so that the required memory capacity and computing costs can be reduced.

Furthermore, the calibration system 100 can be used for a wide range of analyses. For example, the calibration system 100 can be used not only for Raman spectrometry, but also for a wide range of spectrometric methods such as near-infrared spectrometry, infrared spectrometry, ultraviolet spectrometry, fluorescence spectrometry, and visible spectrometry. Furthermore, the calibration system 100 does not necessarily need to involve biological culture, but can be applied even for the process of chemical synthesis or mixing with agitation. The calibration system 100 can also be applied to a measurement target of larger volume without limiting the scale of the measurement target to a scale such as the volume of the non-cultured sample S.

### 2. Configuration of Each Device in Calibration System 100

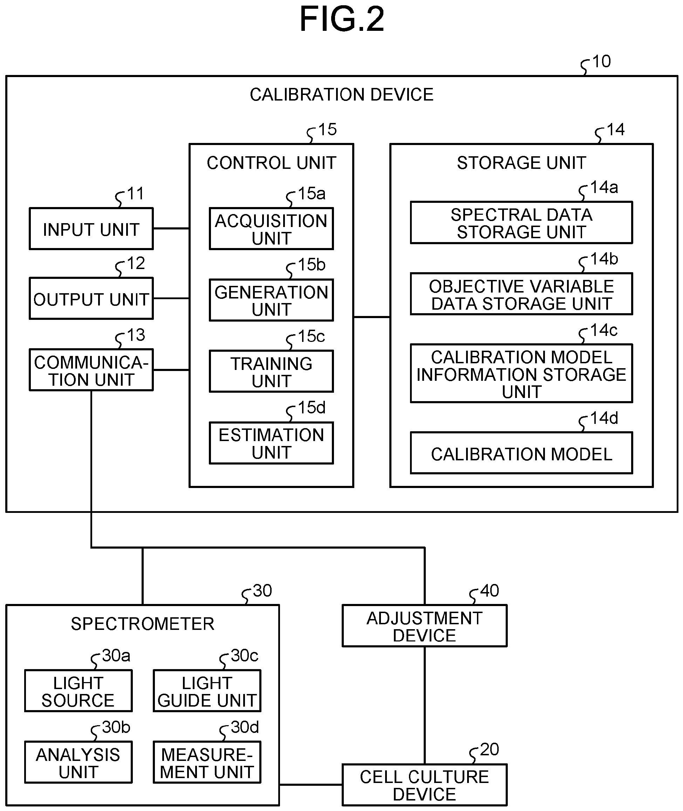

Functional configurations of the devices provided to the calibration system 100 illustrated in Fig. 1 will be described by referring to Fig. 2. Fig. 2 is a block diagram illustrating configuration examples of the devices of the calibration system 100 according to the embodiment. Hereinafter, a configuration example of the calibration device 10, a specific example of an estimation result of the calibration device 10, a configuration example of the cell culture device 20, a configuration example of the spectrometer 30, and a configuration example of the adjustment device 40 will be described in detail in this order.

### 2-1. Configuration Example of Calibration Device 10

First, the configuration example of the calibration device 10 will be described by referring to Fig. 2. The calibration device 10 includes an input unit 11, an output unit 12, a communication unit 13, the storage unit 14, and a control unit 15.

### 2-1-1. Input Unit 11

The input unit 11 controls the input of various kinds of information to the calibration device 10. For example, the input unit 11 is achieved with a mouse, a keyboard, and the like, and accepts input of setting information and the like made onto the calibration device 10.

### 2-1-2. Output Unit 12

The output unit 12 controls output of various information from the calibration device 10. For example, the output unit 12 is achieved by a display or the like, and outputs setting information and the like stored in the calibration device 10.

### 2-1-3. Communication Unit 13

The communication unit 13 controls data communication with other devices. For example, the communication unit 13 performs data communication with each of the communication devices via a router or the like. The communication unit 13 can also perform data communication with a terminal of an operator, not illustrated.

### 2-1-4. Storage Unit 14

The storage unit 14 stores therein various kinds of information that is referred to when the control unit 15 operates, as well as various kinds of information acquired when the control unit 15 operates. For example, the storage unit 14 stores therein the spectral data of a reference sample containing a plurality of components, and each objective variable determined by the content of each of the components of the reference sample. The storage unit 14 includes a spectral data storage unit 14a, an objective variable data storage unit 14b, a calibration model information storage unit 14c, and the calibration model 14d. Note here that the storage unit 14 can be achieved, for example, by a semiconductor memory element such as Random Access Memory (RAM) and a flash memory, or a storage device such as a hard disk, an optical disc, and the like. While the storage unit 14 is placed inside the calibration device 10 in the example of Fig. 2, it may be placed outside the calibration device 10, or a plurality of storage units may be placed as well.

### 2-1-4-1. Spectral Data Storage Unit 14a

The spectral data storage unit 14a stores therein spectral data acquired by an acquisition unit 15a of the control unit 15. For example, the spectral data storage unit 14a stores therein spectral data of the non-cultured sample S acquired from a non-cultured sample measurement device, not illustrated. Furthermore, the spectral data storage unit 14a stores therein spectral data of the cell culture medium of the cell culture device 20 acquired from the spectrometer 30. The spectral data storage unit 14a may also store therein spectral data received by the communication unit 13.

### 2-1-4-2. Objective Variable Data Storage Unit 14b

The objective variable data storage unit 14b stores therein the objective variable data acquired by the acquisition unit 15a of the control unit 15. For example, the objective variable data storage unit 14b stores therein concentration data for each component of the non-cultured sample S and the cell culture medium acquired from an analysis device, not illustrated. The objective variable data storage unit 14b also stores therein the concentration data of each component given when preparing the non-cultured sample S. The objective variable data storage unit 14b may also store therein the concentration data received by the communication unit 13. Furthermore, the objective variable data storage unit 14b stores therein the overall objective variables determined by the content of each component, such as cell density, pH (hydrogen ion index), and osmotic pressure, in addition to the concentration data of each component.

### 2-1-4-3. Calibration Model Information Storage Unit 14c

The calibration model information storage unit 14c stores therein the calibration model information acquired by a training unit 15c of the control unit 15. For example, the calibration model information storage unit 14c stores therein information on spectral preprocessing, a wavenumber range, a regression coefficient of a regression model, signal processing, and the like. The calibration model information storage unit 14c also stores therein, as the calibration model information, a first development condition acquired by using cross-validation on a reference sample data set. Furthermore, the calibration model information storage unit 14c also stores therein, as the calibration model information, a second development condition acquired by using a measurement target sample data set for a validation set.

### 2-1-4-4. Calibration Model 14d

The calibration model 14d is a machine learning model that outputs an objective variable such as the concentration of each component, when spectral data is input. For example, the calibration model 14d is a machine learning model achieved by Partial Least Squares Regression (PLSR), Principal Component Regression, Gaussian Process Regression, or the like.

### 2-1-5. Control Unit 15

The control unit 15 controls the entire calibration device 10. The control unit 15 includes the acquisition unit 15a, a generation unit 15b, the training unit 15c, and an estimation unit 15d. Note here that the control unit 15 can be achieved, for example, by an electronic circuit such as a Central Processing Unit (CPU) or a Micro Processing Unit (MPU), or an integrated circuit such as an Application Specific Integrated Circuit (ASIC) or a Field Programmable Gate Array (FPGA).

In addition, the control unit 15 has a computational environment in which a regression model can be constructed by multivariate analysis. For example, the control unit 15 has a package of a chemometric method and a multivariate analytic method, and an environment for performing calculations using the package. The control unit 15 may also be connectable to a network.

### 2-1-5-1. Acquisition Unit 15a

The acquisition unit 15a acquires spectral data measured by a spectral analysis performed on a reference sample containing a plurality of components. For example, the acquisition unit 15a acquires spectral data for the non-cultured sample S housed in a vessel such as a beaker of several milliliters to several thousand milliliters, using a non-cultured sample measurement device, not illustrated. Furthermore, the acquisition unit 15a acquires spectral data measured by a spectral analysis performed on a sample to be the measurement target. For example, the acquisition unit 15a acquires spectral data measured by Raman spectroscopy, near-infrared spectroscopy, infrared spectroscopy, ultraviolet spectroscopy, fluorescence spectroscopy, visible spectroscopy, or the like using the spectrometer 30. The acquisition unit 15a stores the acquired spectral data in the spectral data storage unit 14a.

### 2-1-5-2. Generation Unit 15b

The generation unit 15b generates a data set of the reference sample including the spectral data of the reference sample containing a plurality of components and each objective variable determined by the content of each of the components of the reference sample. For example, the generation unit 15b acquires, from the storage unit 14, spectral data of a reference sample containing a plurality of components and each objective variable determined by the content of each of the components of the reference sample, and generates a supervised data set of the reference sample.

As for the case of a reference sample containing a plurality of components, the generation unit 15b executes a spectral analysis on a plurality of non-cultured samples with different objective variables determined by the contents of the respective components, and generates a data set of reference samples containing the spectral data of the reference samples acquired by the spectral analysis and each of the objective variables. For example, the generation unit 15b executes Raman spectral analysis on a plurality of non-cultured samples S with different concentrations of glucose and amino acids such as glutamic acid, which are nutrients, and generates a data set of non-cultured samples S including the spectral data of the non-cultured samples S and each of the concentrations. Referring to a specific example, the generation unit 15b executes Raman spectral analysis on a plurality of non-cultured samples S with different concentrations, such as a non-cultured sample S1 {glucose concentration 1.0 g/L, glutamic acid concentration 1.0 g/L}, a non-cultured sample S2 {glucose concentration 2.0 g/L, glutamic acid concentration 2.0 g/L}, and a non-cultured sample S3 {glucose concentration 3.0 g/L, glutamic acid concentration 3.0 g/L}, and generates a data set of non-cultured samples S including the spectral data of the non-cultured samples S1 to S3 and each of the concentrations {glucose concentration, glutamic acid concentration}.

Furthermore, the generation unit 15b executes a spectral analysis on a plurality of non-cultured samples created by using components contained in each of a plurality of cultured samples to be the measurement target, and generates a data set of reference samples including the spectral data of the reference samples acquired by the spectral analysis and each of the objective variables. For example, the generation unit 15b executes Raman spectral analysis on a plurality of non-cultured samples S created by using cultured cells and metabolites to be the measurement target, and generates a data set of the non-cultured samples S including the spectral data of the non-cultured samples S and each of the concentrations. Referring to a specific example, the generation unit 15b executes Raman spectroscopic analysis on a plurality of non-cultured samples S with different components and concentrations, such as a non-cultured sample S11 {cell A concentration 1.0 g/L, antibody A concentration 1.0 g/L}, a non-cultured sample S12 {cell B concentration 2.0 g/L, antibody B concentration 1.0 g/L}, and a non-cultured sample S13 {cell C concentration 1.0 g/L, antibody C concentration 1.0 g/L}, and generates a data set of non-cultured samples S including spectral data of the non-cultured samples S11 to S13 and each of the concentrations {cell concentration, antibody concentration}.

In addition, the generation unit 15b further generates a data set of a sample as the measurement target including spectral data of the sample as the measurement target and each objective variable determined by the content of each of a plurality of components of the sample as the measurement target. For example, the generation unit 15b generates a data set of a cell culture medium including spectral data measured by the spectrometer 30 performing Raman spectrometry on the cell culture medium of the cell culture device 20 and the concentration of each of the components measured by any appropriate analytical method. Referring to a specific example, the generation unit 15b generates a data set of a cell culture medium of a cell A including {Raman spectroscopy spectrum A}, which is spectral data measured by Raman spectrometry performed on the cell culture medium of the cell A as the measurement target, and concentration data {cell A concentration 1.0 g/L, glucose concentration 2.0 g/L, lactic acid concentration 1.0 g/L and antibody A concentration 1.0 g/L}.

While the concentration is described above as an example of the objective variable included in a data set, the objective variable is not limited to the concentration. For example, the generation unit 15b can generate a data set using the overall objective variables determined by the content of each component, such as cell density, pH (hydrogen ion index), and osmotic pressure, in addition to the concentration of each component.

### 2-1-5-3. Training Unit 15c

By machine learning using a data set of a reference sample containing a plurality of components, the training unit 15c trains a machine learning model that outputs at least one objective variable out of the objective variables for each of the components in response to input of the spectral data. For example, by machine learning using a data set of a non-cultured sample S containing a plurality of components, the training unit 15c trains the calibration model 14d that outputs an objective variable that is at least one objective variable out of the objective variables for each of the components in response to input of the spectral data as an explanatory variable. Referring to a specific example, the training unit 15c trains the calibration model 14d by machine learning using a data set 1 {spectral data: Raman spectroscopy spectrum 1, objective variable data: glucose concentration 1.0 g/L, glutamic acid concentration 1.0 g/L}, a data set 2 {spectral data: Raman spectroscopy spectrum 2, objective variable data: glucose concentration 2.0 g/L, glutamic acid concentration 2.0 g/L}, and a data set 3 {spectral data: Raman spectroscopy spectrum 3, objective variable data: glucose concentration 3.0 g/L, glutamic acid concentration 3.0 g/L}.

Furthermore, the training unit 15c searches for a first development condition regarding the algorithm or parameter using cross-validation on the data set of the reference sample containing a plurality of components, trains the machine learning model based on the first development condition, and stores the first development condition in the storage unit 14. For example, the training unit 15c searches for information on spectral preprocessing, a wavenumber range, a regression coefficient of a regression model, signal processing, and the like using cross-validation on a data set of the non-cultured sample S containing a plurality of components, and trains the calibration model 14d based on such information. At this time, the training unit 15c uses the information to specify the optimal algorithm and a parameter of the model, and trains the calibration model 14d with those applied.

The training unit 15c stores, as calibration model information, the searched conditions (information on spectral preprocessing, a wavenumber range, a regression coefficient of the regression model, signal processing, and the like) regarding the algorithm or parameter based on the non-cultured sample S in the calibration model information storage unit 14c.

Furthermore, the training unit 15c searches for a second development condition regarding the algorithm or parameter using the data set of the sample to be the measurement target as a validation set, trains a machine learning model based on the second development condition, and stores the second development condition in the storage unit 14. For example, the training unit 15c searches for the information on spectral preprocessing, a wavenumber range, a regression coefficient of a regression model, signal processing, and the like using, as a validation set, the data set of the cell culture medium of the cell culture device 20, and trains the calibration model 14d based on such information. At this time, the training unit 15c uses the information to specify the optimal algorithm and a parameter of the model, and trains the calibration model 14d with those applied.

The training unit 15c stores, as the calibration model information, the search conditions (information on spectral preprocessing, a wavenumber range, a regression coefficient of the regression model, signal processing, and the like) regarding the algorithm or parameter based on the non-cultured medium of the cell culture device 20 in the calibration model information storage unit 14c.

### 2-1-5-4. Estimation Unit 15d

The estimation unit 15d estimates the objective variable determined by the content of the component contained in the sample as the measurement target, based on the result acquired by inputting the acquired spectral data into a trained machine learning model. For example, the estimation unit 15d estimates the component concentrations of the components contained in the cell culture medium of the cell culture device 20 based on the result acquired by inputting the acquired spectral data of the cell culture medium of the cell culture device 20 into the calibration model 14d. Referring to a specific example, the estimation unit 15d estimates {cell A concentration 2.0 g/L, glucose concentration 1.5 g/L, lactic acid concentration 1.5 g/L, antibody A concentration 2.0 g/L} as the component concentrations of the cell culture medium of the cell A as the measurement target. The estimation unit 15d also estimates, as the process data of the component concentration of the cell culture medium of the cell A as the measurement target, the objective variable at any given time, such as time 1, time 2, time 3, and so on.

Furthermore, the estimation unit 15d can also display the estimation result on the output unit 12. For example, the estimation unit 15d displays, on the output unit 12, a graph indicating the change in the concentration of glucose in the cell culture medium at the time where the spectral data is acquired. Furthermore, the estimation unit 15d displays, on the output unit 12, a graph showing the change in the density of cells in the cell culture medium at the time when the spectral data is acquired.

The estimation unit 15d can also transmit the estimation result to the adjustment device 40. For example, when the concentration of glucose in the cell culture medium falls below a threshold, the estimation unit 15d transmits an instruction to the adjustment device 40 to supply glucose. Furthermore, when the pH of the cell culture medium indicates a value outside the normal range, the estimation unit 15d transmits an instruction to the adjustment device 40 to adjust the pH.

Furthermore, the estimation unit 15d can estimate the objective variable without limiting the scale of the measurement target. For example, the estimation unit 15d can estimate the component concentrations of cell culture medium on the 100-mL-scale, 5-L-scale, 50-L-scale, and 2000-L-scale using the calibration model 14d trained by a data set of the non-cultured samples S on the several-milliliter scale.

### 2-2. Specific Examples of Estimation Result of Calibration Device 10

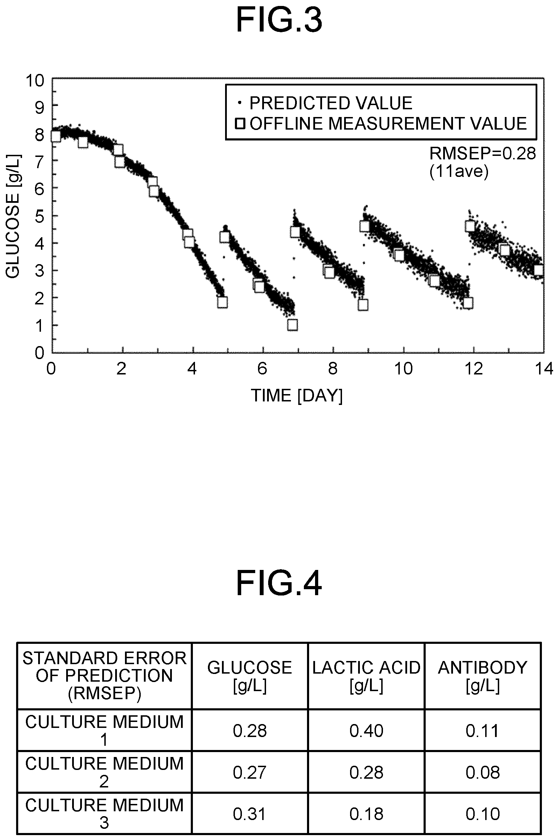

Specific examples of the estimation result of the calibration device 10 will be described by referring to Fig. 3 to Fig. 5. Fig. 3 to Fig. 5 are diagrams illustrating examples of the estimation result according to the embodiment. Hereinafter, Specific Example 1, in which errors in the estimation result are evaluated, and Specific Example 2, in which the conditions of the cell culture medium are significantly changed, will be described.

### 2-2-1. Specific Example 1

Specific Example 1 of the estimation result of the calibration device 10 will be described by referring to Fig. 3 and Fig. 4. In Specific Example 1, nutrient and metabolite concentrations in the cell culture process are predicted by using the trained calibration model 14d. At this time, to grasp the performance of the model, three batches of 5-L-scale cell culture process (culture media 1 to 3) with the same conditions were used.

In the example of Fig. 3, the horizontal axis is "time [day]" and the vertical axis is "glucose [g/L]", showing changes, over time, of the concentration of glucose as a nutrient. Note here that the black dots in Fig. 3 indicate the predicted values that are the estimation result of the calibration device 10. Furthermore, the white squares in Fig. 3 indicate offline measurement values taken to verify the estimation result of the calibration device 10.

The example in Fig. 4 indicates the standard errors of prediction (root mean squared error of prediction: RMSEP) for each of glucose, lactic acid, and antibody in each of the culture media 1 to 3. Note here that the prediction standard error in Fig. 4 is the error calculated by averaging the prediction values of 11 points near the offline measurement values. As in Fig. 4, the standard error of prediction for the culture medium 1 is calculated as {glucose: 0.28 g/L, lactic acid: 0.40 g/L, antibody: 0.11 g/L}, the standard error of prediction for the culture medium 2 as {glucose: 0.27 g/L, lactic acid: 0.28 g/L, antibody: 0.08 g/L}, and the standard error of prediction for the culture medium 3 as {glucose: 0. 31 g/L, lactic acid: 0.18 g/L, antibody: 0.10 g/L}.

From Specific Example 1 described above, the accuracy of the prediction ability of the calibration model 14d and its reproducibility can be confirmed from the results of three batches of the cell culture process.

### 2-2-2. Specific Example 2

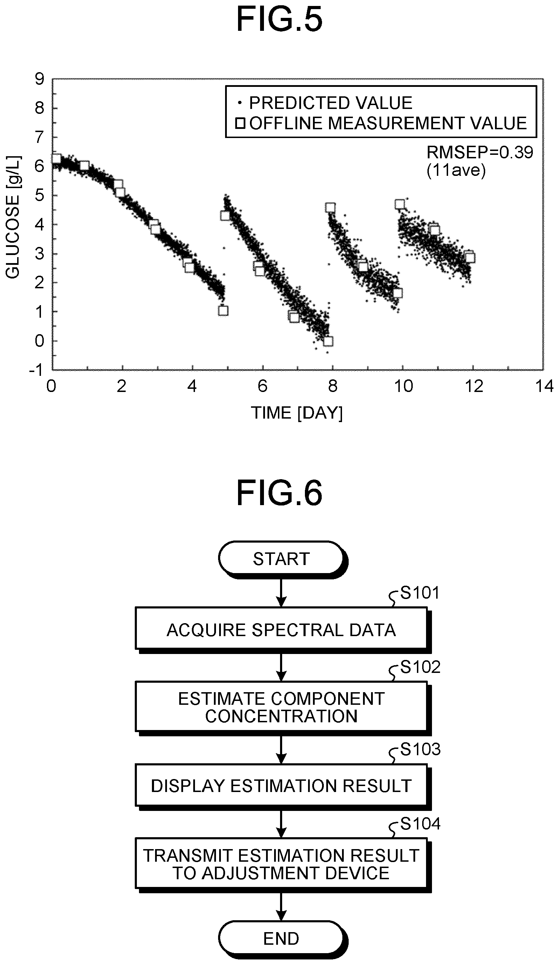

Specific Example 2 of the estimation result of the calibration device 10 will be described by referring to Fig. 5. Specific Example 2 indicates the estimation result when the calibration model 14d is applied to a 5-L-scale cell culture process with conditions that differ significantly from the conditions at the time of generating the model, that is, with a changed culture medium, and, as in Specific Example 1, indicates prediction of the concentration of nutrients and metabolites in the cell culture process.

In the example of Fig. 5, as in Fig. 3, the horizontal axis is "time [day]" and the vertical axis is "glucose [g/L]", showing changes, over time, of the concentration of glucose as a nutrient. Here, the black dots in Fig. 5 indicate the predicted values that are the estimation result of the calibration device 10. Furthermore, the white squares in Fig. 5 indicate offline measurement values taken to verify the estimation result of the calibration device 10.

From Specific Example 2 described above, it can be confirmed that the concentrations of nutrients and metabolite components can be predicted also in cell culture under different conditions.

### 2-3. Configuration Example of Cell Culture Device 20

The configuration example of the cell culture device 20 illustrated in Fig. 1 will be described by referring to Fig. 2 The cell culture device 20 includes a cell culture tank, not illustrated. The cell culture tank houses therein a cell culture medium containing cells, nutrient components including glucose and amino acids, and cell metabolites. Here, as cultures cultured by the cell culture medium, microorganisms, yeast, and the like may be used in addition to cells.

### 2-4. Configuration Example of Spectrometer 30

The configuration example of the spectrometer 30 illustrated in Fig. 1 will be described by referring to Fig. 2. The spectrometer 30 includes a light source 30a, an analysis unit 30b, a light guide unit 30c, and a measurement unit 30d.

### 2-4-1. Light Source 30a

The light source 30a is a source of light applied to the cell culture medium. The light source 30a is a source of light used for Raman spectroscopy, near-infrared spectroscopy, infrared spectroscopy, ultraviolet spectroscopy, fluorescence spectroscopy, visible spectroscopy, and the like. The type and wavelength of light from the light source 30a can be changed depending on the purpose of the measurement.

### 2-4-2. Analysis Unit 30b

The analysis unit 30b analyzes the light guided from the measurement unit 30d via the light guide unit 30c. For example, the analysis unit 30b selects the optimal spectroscopic means depending on the purpose of the measurement, and analyzes the guided light. The analysis unit 30b also includes spectroscopic means, and detects the intensity of each wavelength of light to calculate the spectrum.

### 2-4-3. Light Guide Unit 30c

The light guide unit 30c guides the light from the light source 30a to the measurement unit 30d. The light guide unit 30c also guides the light reflected from the cell culture medium to the analysis unit 30b. For example, the light guide unit 30c can be achieved by a mirror, an optical fiber, a lens, or the like.

### 2-4-4. Measurement Unit 30d

The measurement unit 30d applies light onto the cell culture medium as the measurement target, and receives the reflected light or scattered light. Furthermore, the measurement unit 30d has a structure such that the culture medium, which is sufficiently agitated and homogenized without affecting the cell culture, is exposed to the incident light. For example, it can also be achieved by a focusing lens probe, a flow cell, or the like.

### 2-5. Configuration Example of Adjustment Device 40

The configuration example of the adjustment device 40 illustrated in Fig. 1 will be described by referring to Fig. 2. The adjustment device 40 adjusts the environment of the cell culture tank (temperature, pH, dissolved oxygen, nutrient concentration, and the like). For example, the adjustment device 40 can be achieved by a pump or a heater that supplies nutrients.

### 3. Flows of Processing of Calibration System 100

The flows of the processing of the calibration system 100 according to the embodiment will be described by referring to Fig. 6 to Fig. 8. Hereinafter, the flow of data estimation processing, the flow of calibration model construction processing 1, and the flow of calibration model construction processing 2 will be described in this order.

### 3-1. Flow of Data Estimation Processing

The flow of the data estimation processing according to the embodiment will be described by referring to Fig. 6. Fig. 6 is a flowchart illustrating an example of the flow of the data estimation processing according to the embodiment. Note that the processing of the following steps S101 to S104 may also be executed in a different order. Furthermore, some of the processing of the following steps S101 to S104 may be omitted.

### 3-1-1. Spectral Data Acquisition Processing

First, the acquisition unit 15a of the calibration device 10 acquires spectral data (step S101). For example, the acquisition unit 15a acquires spectral data of the cell culture medium measured by Raman spectroscopy, near-infrared spectroscopy, infrared spectroscopy, ultraviolet spectroscopy, fluorescence spectroscopy, visible spectroscopy, or the like using the spectrometer 30. At this time, the acquisition unit 15a acquires the data converted into digital information by the analysis unit 30b of the spectrometer 30, and calculates the spectral data from the data.

### 3-1-2. Objective Variable Estimation Processing

Secondly, the estimation unit 15d of the calibration device 10 estimates the objective variable such as component concentration (step S102). For example, the estimation unit 15d estimates the concentration of each component such as glucose contained in the cell culture medium of the cell culture device 20 based on the result acquired by inputting the acquired spectral data of the cell culture medium of the cell culture device 20 into the calibration model 14d. At this time, the estimation unit 15d applies the acquired spectral data to the calibration model information read out from the storage unit 14 to calculate the predicted value of the amount of the measurement target component (e.g., process variable such as nutrient concentration value) from the peak intensity.

### 3-1-3. Estimation Result Display Processing

Thirdly, the estimation unit 15d of the calibration device 10 displays the estimation result (step S103). For example, the estimation unit 15d displays, on the output unit 12, a graph indicating the change in the concentration of glucose in the cell culture medium at the time when the spectral data is acquired.

### 3-1-4. Estimation Result Transmission Processing

Fourthly, the estimation unit 15d of the calibration device 10 transmits the estimation result (step S104), and ends the processing. For example, when the concentration of glucose in the cell culture medium falls below a threshold, the estimation unit 15d transmits an instruction to the adjustment device 40 to supply glucose. At this time, the adjustment device 40 that has received the estimation result executes optimization of the cell culture tank of the cell culture device 20.

### 3-1-5. Effects of Data Estimation Processing

In the data estimation processing described above, the concentration of the target component can be estimated in real time by processing the spectral data acquired by connecting the spectrometer 30 to the cell culture device 20 with the calibration model 14d. Furthermore, with the data estimation processing, the optimal culture environment can be maintained by controlling the concentration in the culture medium in real time by using the estimated value of the concentration of the target component.

### 3-2. Flow of Calibration Model Construction Processing 1

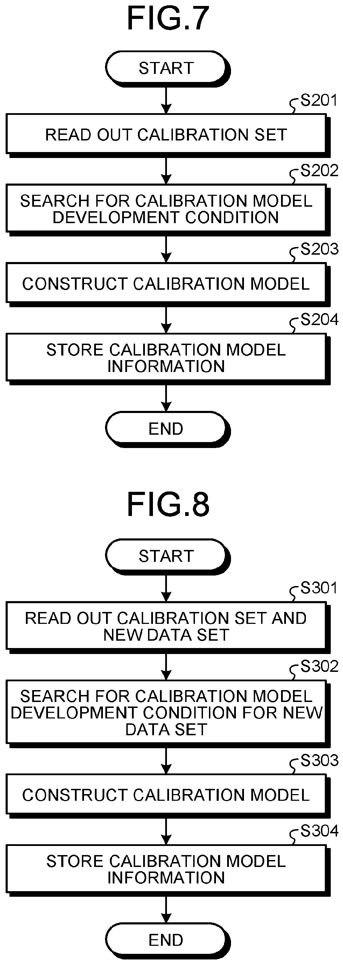

The flow of the calibration model construction processing 1 according to the embodiment will be described by referring to Fig. 7. Fig. 7 is a flowchart illustrating an example of the flow of the calibration model construction processing 1 according to the embodiment. Note that the processing of the following steps S201 to S204 may also be executed in a different order. Furthermore, some of the processing of the following steps S201 to S204 may be omitted.

### 3-2-1. Data Set Acquisition Processing

First, the generation unit 15b of the calibration device 10 reads out a calibration set (step S201). For example, the generation unit 15b acquires the spectral data measured on the non-cultured sample S prior to the culture process prediction and the objective variable data from the storage unit 14, and generates a supervised data set. At this time, the generation unit 15b may read out a calibration set stored in the storage unit 14 as a supervised data set.

### 3-2-2. Development Condition Search Processing

Secondly, the training unit 15c of the calibration device 10 searches for the development conditions for the calibration model 14d (step S202). For example, the training unit 15c uses cross-validation on the data set of the non-cultured sample S for searching for the first development condition (information on spectral preprocessing, a wavenumber range, a regression coefficient of the regression model, signal processing, and the like acquired from the data set of the non-cultured sample S) regarding the type of algorithm and a parameter of the analysis.

### 3-2-3. Calibration Model Training Processing

Thirdly, the training unit 15c of the calibration device 10 constructs the calibration model 14d based on the search result (step S203). For example, by machine learning using a data set of the non-cultured sample S, the training unit 15c trains the calibration model 14d that outputs the component concentration as an objective variable in response to input of spectral data as an explanatory variable.

### 3-2-4. Calibration Model Information Storage Processing

Fourthly, the training unit 15c of the calibration device 10 stores the calibration model information (step S204), and ends the processing. At this time, the training unit 15c stores information on spectral preprocessing, a wavenumber range, a regression coefficient of the regression model, signal processing, and the like in the calibration model information storage unit 14c.

### 3-2-5. Effects of Calibration Model Construction Processing 1

The calibration model construction processing 1 described above does not require cell culture with sampling for preparing a calibration set, so that the time and cost for creating a model can be reduced significantly and the risk of having loss and contamination of the culture medium can be eliminated. In addition, the calibration model construction processing 1 improves the robustness of the quantitative model for the cell culture conditions, thereby making it possible to be applied to various culture conditions. Furthermore, the calibration model construction processing 1 can reduce the number of data pieces in a calibration set compared to the conventional methods, so that the required memory capacity and computing costs can be reduced.

### 3-3. Flow of Calibration Model Construction Processing 2

Furthermore, the flow of the calibration model construction processing 2 according to the embodiment, which further uses a newly acquired data set by operating the cell culture device 20, will be described by referring to Fig. 8. Fig. 8 is a flowchart illustrating an example of the flow of the calibration model construction processing 2 according to the embodiment. Note that the processing of the following steps S301 to S304 may also be executed in a different order. Furthermore, some of the processing of the following steps S301 to S304 may be omitted.

### 3-3-1. Data Set Acquisition Processing

First, the generation unit 15b of the calibration device 10 reads out a calibration set and a newly acquired data set (step S301). At this time, as spectral data, the generation unit 15b uses the data acquired by connecting to the cell culture device 20 for the newly acquired data set. Alternatively, the generation unit 15b may use measurement data acquired by another spectrometer. Furthermore, as objective variable data, the generation unit 15b uses data obtained by any appropriate analysis method by performing sampling before and after the measurement of the spectral data.

For example, as a calibration set, the generation unit 15b reads out a data set of the non-cultured sample S saved in advance in the storage unit 14. The generation unit 15b also reads out the 5-L-scale cell culture data as a newly acquired data set. At this time, as the cell culture data for the newly acquired data set, the generation unit 15b may use the same or different culture medium components as or from those of the non-cultured sample S. Note that the above culture media are used primarily for dilution of the non-cultured sample S, and the contents thereof vary for each product of manufactures.

### 3-3-2. Development Condition Search Processing

Secondly, the training unit 15c of the calibration device 10 searches for the development conditions for the calibration model 14d (step S302). At this time, the training unit 15c uses the newly acquired data set as a validation set to search for the second development condition (information on spectral preprocessing, a wavenumber range, a regression coefficient of the regression model, signal processing, and the like acquired from the newly acquired data set) regarding the type of algorithm and a parameter of the analysis.

### 3-3-3. Calibration Model Training Processing

Thirdly, the training unit 15c of the calibration device 10 constructs the calibration model 14d based on the search result (step S303). Since the calibration model training processing is the same as the calibration model training processing of the above-described calibration model construction processing 1, the explanation thereof is omitted.

### 3-3-4. Calibration Model Information Storage Processing

Fourthly, the training unit 15c of the calibration device 10 stores the calibration model information (step S304), and ends the processing. Since the calibration model information storage processing is the same as the calibration model information storage processing of the above-described calibration model construction processing 1, the explanation thereof is omitted.

### 3-3-5. Evaluation of Calibration Model Construction Processing 2

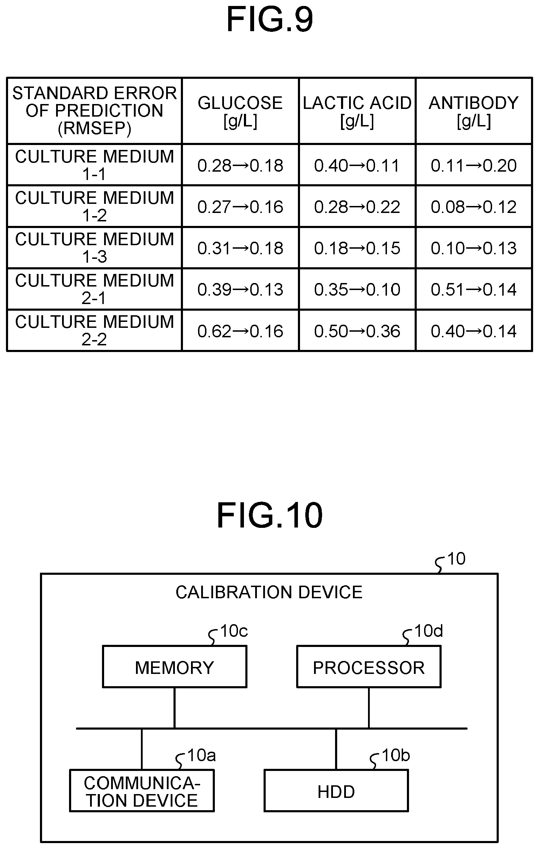

Evaluation of the calibration model construction processing 2 will be described by referring to Fig. 9. Fig. 9 is a table illustrating an example of the evaluation of the calibration model construction processing 2 according to the embodiment. The example in Fig. 9 indicates the change in the standard errors of prediction (RMSEP) for each of glucose, lactic acid, and antibody in the culture media 1-1 to 1-3 and the culture media 2-1 to 2-2. Note here that the culture media 1-1 to 1-3 are 5-L-scale cell culture media under the same conditions as those when the model is created, and the culture media 2-1 to 2-2 are 5-L-scale cell culture media under significantly different conditions compared to those when the model is created, that is, 5-L-scale cell culture media with a changed culture medium.

As in Fig. 9, the standard errors of prediction for the culture medium 1-1 are calculated as {glucose: 0.28 g/L → 0.18 g/L, lactic acid: 0.40 g/L → 0.11 g/L, antibody: 0.11 g/L → 0.20 g/L}, the standard errors of prediction for the culture medium 1-2 as {glucose: 0.27 g/L → 0.16 g/L, lactic acid: 0.28 g/L → 0.22 g/L, antibody: 0.08 g/L → 0.12 g/L}, and the standard errors of prediction for the culture medium 1-3 as {glucose: 0.31 g/L → 0.18 g/L, lactic acid: 0.18 g/L → 0.15 g/L, antibody: 0.10 g/L → 0.13 g/L}. Furthermore, the standard errors of prediction for the culture medium 2-1 are calculated as {glucose: 0.39 g/L→ 0.13 g/L, lactic acid: 0.35 g/L → 0.10 g/L, antibody: 0.51 g/L → 0.14 g/L}, and the standard errors of prediction for the culture medium 2-2 as {glucose: 0.62 g/L → 0.16 g/L, lactic acid: 0.50 g/L → 0.36 g/L, antibody: 0.40 g/L → 0.14 g/L}.

As described above, it is possible to confirm the improvement in calibration accuracy of the calibration model 14d constructed by the calibration model construction processing 2.

### 3-3-6. Effects of Calibration Model Construction Processing 2

The calibration model construction processing 2 can create a new model by using a data set of cell culture data of one or more times for validation for the calibration set containing only the non-cultured spectra. In other words, in addition to the effects of the calibration model construction processing 1, it is possible with the calibration model construction processing 2 to match the various conditions of the calibration model 14d appropriate for the measurement target to be actually predicted by using the newly acquired data set for validation, thereby making it possible to improve the calibration accuracy.

### 4. Effects of Embodiment

Finally, effects of the embodiment will be described. Hereinafter, effects 1 to 6 corresponding to the processing according to the embodiment will be described.

### 4-1. Effect 1

First, in the processing according to the embodiment, a reference sample data set including spectral data of a reference sample containing a plurality of components and each objective variable determined by the content of each of the components of the reference sample is generated, and, by machine learning using the reference sample data set, the calibration model 14d that outputs at least one objective variable among the objective variables for each of the components is trained in response to input of the spectral data. Therefore, with the processing according to the embodiment, it is possible to create a highly accurate calibration model 14d in an effective manner.

### 4-2. Effect 2

Secondly, in the processing according to the embodiment, a spectral analysis is executed on a plurality of non-cultured samples with different objective variables for each of the components, and a data set of reference samples containing the spectral data of the reference sample acquired by the spectral analysis and each of the objective variables is generated. Therefore, with the processing according to the embodiment, it is possible to create a highly accurate calibration model 14d in an effective manner by using the reference samples with different objective variables.

### 4-3. Effect 3

Thirdly, in the processing according to the embodiment, a spectral analysis is executed on a plurality of non-cultured samples created by using components contained in each of a plurality of cultured samples to be the measurement target, and a data set of reference samples containing the spectral data of the reference samples acquired by the spectral analysis and each of the objective variables is generated. Therefore, with the processing according to the embodiment, it is possible to create a highly accurate calibration model 14d in an effective manner by using the reference samples having similar components as those of the cultured samples to be the measurement target.

### 4-4. Effect 4

Fourthly, in the processing according to the embodiment, spectral data measured by a spectral analysis performed on the sample as the measurement target is acquired, and the objective variable determined by the content of the components contained in the sample as the measurement target is estimated based on the result acquired by inputting the acquired spectral data into the trained calibration model 14d. Therefore, with the processing according to the embodiment, it is possible to create a highly accurate calibration model 14d in an effective manner and to optimize the environment of the measurement target by utilizing the estimation result.

### 4-5. Effect 5

Fifthly, in the processing according to the embodiment, spectral data of a reference sample and each of the objective variables are held; the held spectral data of the reference sample and each of the objective variables are acquired; a supervised data set of the reference sample is generated; cross-validation on the data set of the reference sample is used to search for the development condition regarding the algorithm or parameter; a machine learning model is trained based on the development condition; and the development condition is held. Therefore, with the processing according to the embodiment, it is possible to create a highly accurate calibration model 14d in an effective manner by using offline data of the reference sample and by performing an appropriate adjustment of the calibration model 14d.

### 4-6. Effect 6

Sixthly, in the processing according to the embodiment, a data set of a measurement target sample including the spectral data of the measurement target sample and each objective variable determined by the content of each of the components of the measurement target sample is further generated; the data set of the measurement target sample is used for a validation set to further search for the development condition regarding the algorithm or parameter; and the calibration model 14d is trained based on the development condition. Therefore, with the processing according to the embodiment, it is possible to create a highly accurate calibration model in an effective manner by performing an appropriate adjustment of the learning model in accordance with the measurement target actually desired to predict.

### System

Note that the processing procedures, control procedures, specific names, and information including various kinds of data and parameters discussed in the above and the drawings can be changed as desired, unless otherwise noted.

Furthermore, each of the structural elements of each of the devices in the drawings is illustrated as a functional concept and does not necessarily need to be physically configured as illustrated in the drawings. In other words, the specific forms of distribution and integration of the devices are not limited to those illustrated in the drawings. That is, all or some thereof may be functionally or physically distributed or integrated in arbitrary units according to various kinds of loads, usage conditions, and the like.

Furthermore, all or some of the processing functions performed by each of the devices can be achieved by a CPU and a computer program analyzed and executed by the CPU or can be achieved as hardware using wired logic.

### Hardware

Next, an example of the hardware configuration of the calibration device 10 will be described. Fig. 10 is a diagram for describing an example of the hardware configuration. As illustrated in Fig. 10, the calibration device 10 includes a communication device 10a, a hard disk drive (HDD) 10b, a memory 10c, and a processor 10d. Furthermore, each of the units illustrated in Fig. 10 is interconnected by a bus or the like.

The communication device 10a is a network interface card or the like, and communicates with other servers. The HDD 10b stores therein computer programs and DBs for operating the functions illustrated in Fig. 2.

The processor 10d reads out a computer program for executing the same processing as that of each of the processing units illustrated in Fig. 2 from the HDD 10b or the like, and loading it on the memory 10c so as to operate the process for executing each of the functions described by referring to Fig. 2 and the like. For example, in the process, the same function as that of each of the processing units of the calibration device 10 is executed. Specifically, the processor 10d reads out the computer program having the same functions as those of the acquisition unit 15a, the generation unit 15b, the training unit 15c, the estimation unit 15d, and the like from the HDD 10b or the like. Then, the processor 10d executes the process for executing the same processing as those of the acquisition unit 15a, the generation unit 15b, the training unit 15c, the estimation unit 15d, and the like.

As described, the calibration device 10 operates as a device that executes various kinds of processing methods by reading out and executing the computer program. Furthermore, the calibration device 10 can also achieve the same functions as those of the embodiment described above by reading out the computer program from a recording medium using a medium reading device and executing the read-out program. Note that other computer programs in the embodiment are not necessarily executed by the calibration device 10. For example, the present invention can also be applied in a similar manner to cases where another computer or server executes the computer program or where these execute the computer program in cooperation.

The computer program can be distributed via a network such as the Internet. The computer program can also be recorded on a computer-readable recording medium such as a hard disk, a flexible disk (FD), a CD-ROM, an MO (Magneto-Optical disk), or a DVD (Digital Versatile Disc), and executed by a computer reading it out from the recording medium.

### Others

Some examples of combinations of the disclosed technical features will be described hereinafter.
(1) A calibration device, including: a generation unit that generates a data set of a reference sample including spectral data of the reference sample containing a plurality of components and each objective variable determined by a content of each of the components of the reference sample; and a training unit that trains, by machine learning using the data set of the reference sample, a machine learning model that outputs at least one objective variable among the objective variables of each of the components in response to input of the spectral data.
(2) The calibration device according to (1), in which the generation unit: executes a spectral analysis on a plurality of non-cultured samples having different objective variables determined by contents of the respective components; and generates a data set of the reference sample including the spectral data of the reference sample acquired by the spectral analysis and each of the objective variables.
(3) The calibration device according to (2), in which the generation unit: executes a spectral analysis on the non-cultured samples created by using components contained in each of a plurality of cultured samples to be a measurement target; and generates a data set of the reference sample including the spectral data of the reference sample acquired by the spectral analysis and each of the objective variables.
(4) The calibration device according to any one of (1) to (3), further including: an acquisition unit that acquires spectral data measured by a spectral analysis performed on a sample to be a measurement target; and an estimation unit that estimates an objective variable determined by the content of a component contained in the sample to be the measurement target, based on a result acquired by inputting the acquired spectral data into the machine learning model that has been trained.
(5) The calibration device according to (1) to (4), further including a storage unit that stores therein the spectral data of the reference sample and each of the objective variables, in which the generation unit: acquires the spectral data of the reference sample and each of the objective variables from the storage unit; and generates a supervised data set of the reference sample, and the training unit: searches for a first development condition regarding an algorithm or a parameter using cross-validation on the data set of the reference sample; trains the machine learning model based on the first development condition; and stores the first development condition in the storage unit.
(6) The calibration device according to (5), in which the generation unit further generates a data set of a sample to be the measurement target including spectral data of the sample to be the measurement target and each objective variable determined by the content of each of the components of the sample to be the measurement target, and the training unit: further searches for a second development condition regarding the algorithm or the parameter by using the data set of the sample to be the measurement target as a validation set; trains the machine learning model based on the second development condition; and stores the second development condition in the storage unit.
(7) A calibration method including: generating a data set of a reference sample including spectral data of the reference sample containing a plurality of components and each objective variable determined by a content of each of the components of the reference sample; and training, by machine learning using the data set of the reference sample, a machine learning model that outputs at least one objective variable among the objective variables of each of the components in response to input of the spectral data.
(8) A calibration program that causes a computer to execute a process including: generating a data set of a reference sample including spectral data of the reference sample containing a plurality of components and each objective variable determined by a content of each of the components of the reference sample; and training, by machine learning using the data set of the reference sample, a machine learning model that outputs at least one objective variable among the objective variables of each of the components in response to input of the spectral data.

According to the present invention, it is possible to create a highly accurate calibration model in an effective manner.

## Claims

1. A calibration device (10), comprising:
a generation unit (15b) that generates a data set of a reference sample including spectral data of the reference sample containing a plurality of components and each objective variable determined by a content of each of the components of the reference sample; and
a training unit (15c) that trains, by machine learning using the data set of the reference sample, a machine learning model (14d) that outputs at least one objective variable among the objective variables of each of the components in response to input of the spectral data.

2. The calibration device (10) according to claim 1, wherein
the generation unit (15b):
executes a spectral analysis on a plurality of non-cultured samples having different objective variables determined by the contents of the components; and
generates a data set of the reference sample including the spectral data of the reference sample acquired by the spectral analysis and each of the objective variables.

3. The calibration device (10) according to claim 2, wherein
the generation unit (15b):
executes a spectral analysis on the non-cultured samples created by using components contained in each of a plurality of cultured samples to be a measurement target; and
generates a data set of the reference sample including the spectral data of the reference sample acquired by the spectral analysis and each of the objective variables.

4. The calibration device (10) according to any one of claims 1 to 3, further including:
an acquisition unit (15a) that acquires spectral data measured by a spectral analysis performed on a sample to be a measurement target; and
an estimation unit (15d) that estimates an objective variable determined by the content of a component contained in the sample to be the measurement target, based on a result acquired by inputting the acquired spectral data into the machine learning model (14d) that has been trained.

5. The calibration device (10) according to claim 4, further including a storage unit (14) that stores therein the spectral data of the reference sample and each of the objective variables, wherein
the generation unit (15b): acquires the spectral data of the reference sample and each of the objective variables from the storage unit (14); and generates a supervised data set of the reference sample, and
the training unit (15c): searches for a first development condition regarding an algorithm or a parameter using cross-validation on the data set of the reference sample; trains the machine learning model (14d) based on the first development condition; and stores the first development condition in the storage unit (14).

6. The calibration device (10) according to claim 5, wherein the generation unit (15b) further generates a data set of a sample to be the measurement target including spectral data of the sample to be the measurement target and each objective variable determined by the content of each of the components of the sample to be the measurement target, and the training unit (15c): further searches for a second development condition regarding the algorithm or the parameter by using the data set of the sample to be the measurement target as a validation set; trains the machine learning model (14d) based on the second development condition; and stores the second development condition in the storage unit (14).

7. A calibration method comprising:
generating a data set of a reference sample including spectral data of the reference sample containing a plurality of components and each objective variable determined by a content of each of the components of the reference sample; and
training, by machine learning using the data set of the reference sample, a machine learning model that outputs at least one objective variable among the objective variables of each of the components in response to input of the spectral data.

8. A calibration program that causes a computer (10) to execute a process comprising:
generating a data set of a reference sample including spectral data of the reference sample containing a plurality of components and each objective variable determined by a content of each of the components of the reference sample; and
training, by machine learning using the data set of the reference sample, a machine learning model that outputs at least one objective variable among the objective variables of each of the components in response to input of the spectral data.
